**Europäisches Patentamt**

**European Patent Office**

**Office européen des brevets**

(19)

(11) Veröffentlichungsnummer : **0 139 863**
**B1**

(12) **EUROPÄISCHE PATENTSCHRIFT**

(45) Veröffentlichungstag der Patentschrift :
09.09.87

(51) Int. Cl.⁴ : **A 61 F  5/449**, A 61 L 25/00

(21) Anmeldenummer : **84108168.0**

(22) Anmeldetag : **12.07.84**

(54) Selbstklebend ausgerüstete Vorrichtung für medizinische Zwecke.

(30) Priorität : **06.08.83 DE 3328433**

(43) Veröffentlichungstag der Anmeldung :
**08.05.85 Patentblatt 85/19**

(45) Bekanntmachung des Hinweises auf die Patenterteilung : **09.09.87 Patentblatt 87/37**

(84) Benannte Vertragsstaaten :
**DE FR GB IT NL SE**

(56) Entgegenhaltungen :
**CH-A-   638 392**
**DE-A- 2 449 135**

(73) Patentinhaber : **Beiersdorf Aktiengesellschaft**
**Unnastrasse 48**
**D-2000 Hamburg 20 (DE)**

(72) Erfinder : **Schulte, Dietrich, Dr. Dipl.-Chem.**
**Reichenberger Strasse 11**
**D-2080 Pinneberg (DE)**
Erfinder : **Sinnen, Marike**
**Fröbelstrasse 7**
**D-2080 Pinneberg (DE)**

## Beschreibung

Die Erfindung betrifft eine selbstklebend ausgerüstete Vorrichtung in Blattform mit einer vorzugsweise mittigen Öffnung, welche zur Befestigung einer chirurgischen, nachoperativen Drainagevorrichtung, insbesondere von Kolostomie-, Ileostomie- oder Ureterostomiebeuteln, auf der Haut dient.

Derartige Drainagevorrichtungen zum Auffangen der Körperausscheidungen bestehen üblicherweise aus einem flüssigkeits- und geruchsdichten Folienbeutel mit einer Öffnung für das Stoma und werden mit Hilfe eines Tragegürtels oder einem Selbstklebeetikett auf der Haut um das Stoma, die künstliche Körperöffnung, herum angebracht. Da die Beutel z. T. mehrmals am Tage ausgewechselt werden müssen, wird die Haut im Verklebungsbereich stark beansprucht und zeigt besonders bei sehr vernarbtem Gewebe oftmals entzündliche Reizerscheinungen. Man ist deshalb in solchen Fällen dazu übergegangen, den Beutel nicht mehr direkt auf der Haut aufzukleben, sondern um das Stoma eine sog. Hautschutzfolie anzubringen, welche mehrere Tage nicht ausgewechselt zu werden braucht, und auf diese Folie den Beutel aufzukleben.

Diese Hautschutzfolien, wie etwa gemäß der DE-A-28 25 195, sind runde bis viereckige Etiketten mit einer Seitenlänge von etwa 10-12 cm und einem mittigen Loch entsprechend der Größe des Stoma. Sie bestehen aus einer relativ dicken hautfreundlichen selbstklebenden Schicht auf einer dünnen Folie, meist aus Polyethylen, als Trägermaterial.

Durch die Verwendung einer derartigen Dauerklebefläche, die etwa 7 Tage aufgeklebt gelassen werden kann, wird zwar die Haut vor der dauernden Reizung durch das häufige Abziehen und Wiederanheften eines Beutels bewahrt. Diese Klebeflächen weisen jedoch den Nachteil auf, daß sie nicht luft- und wasserdampfdurchlässig sind und deshalb die darunter luftdicht abgeschlossene Haut zur Mazeration neigt. Ein Perforieren der Flächen würde keine Abhilfe bringen, da die Poren durch die darüber aufzubringende Klebefläche des Beutels wieder verschlossen würden.

Aus der DE-A-2 449 135 ist zwar bereits die Verwendung eines porösen, luftdurchlässigen Folienmaterials, das ein- oder beidseitig mit einer Selbstklebemasse örtlich beschichtet ist, als Haftplatten für Ostomiebeutel oder dgl. bekannt. Diese sind jedoch mit dem Beutel fest verbunden und müssen deshalb mit jedem gefüllten und damit verbrauchten Beutel weggeworfen werden.

Aufgabe der Erfindung war es deshalb die mehrfach verwendbaren Hautschutzfolien so zu verbessern, daß deren Vorteile, nämlich die leichte Auswechselbarkeit des Auffangbeutels, erhalten bleibt, und die Nachteile, die durch das luftdichte Verschließen der Haut entstehen, vermieden werden.

Die Aufgabe wird gelöst durch eine Vorrichtung der eingangs genannten Art, welche aus einem porösen, luft- und wasserdampfdurchlässigen Trägermaterial besteht, das auf der einen Seite mit einer Selbstklebemasse porös beschichtet ist, die dadurch gekennzeichnet ist, daß sie auf der anderen (der mit Selbstklebemasse versehenen, gegenüberliegenden) Seite mit einer etwa gleich großen Folie abgedeckt und mit dieser stellenweise, in unterbrochener Anordnung fest verbunden ist. Die Verbindungsstellen dürfen dabei insgesamt etwa 5-60 %, vorzugsweise 10-20 %, der Fläche der Vorrichtung ausmachen.

Die Vorrichtung kann analog den bekannten Dauerklebeplatten längere Zeit auf der Haut belassen und der auf die Folie zu klebende Auffangbeutel dabei beliebig oft ausgewechselt werden, gleichzeitig kann aber auch die darunter befindliche Haut durch das poröse Trägermaterial und durch die sich zwischen diesem Trägermaterial und der Folie aufgrund der nur punktuellen Verbindung befindlichen freien Kanäle « atmen ».

Als Trägermaterial lassen sich die für die Pflasterherstellung bekannten Materialien verwenden, sofern sie nur genügend luft- und wasserdampfdurchlässig sind, d. h. insbesondere Gewebe, perforierte Folien und Vliese auf Natur- oder Kunststoffbasis. Vorzugsweise eignen sich hierfür die nach dem sog. « spun-laced » Verfahren hergestellten, besonders luftdurchlässigen Polyester-Vliese (Sontara®-Vliese der Fa. Dupont).

Das Trägermaterial wird auf seiner der Haut zugewandten Seite mit einer der üblichen gut haftenden Selbstklebemassen beschichtet auf Basis Kautschuk/Harz oder synthetischen Polymeren, vorzugsweise Acrylsäureesterpolymere oder -copolymere, welche zusätzlich noch hydrophile Gruppen und/oder hydrophile Substanzen wie Hydrokolloide enthalten können. Die Klebeschichten werden in an sich bekannter Weise, beispielsweise nach dem Verfahren gemäß der DE-C-15 69 901, porös bzw. mikroporös aufgebracht und bis zum Gebrauch der Vorrichtung mit einem leicht wieder abziehbaren Schutzblatt aus beispielsweise silikonisiertem Papier abgedeckt. Eine Schlitzung dieses Abdeckpapiers erleichtert das Abziehen.

Als Abdeckfolie können alle Folienmaterialien verwendet werden, auf denen die üblichen Selbstklebemassen auf den Klebeetiketten der Auffangbeutel gut haften und von denen sie sich auch wieder problemlos abziehen lassen, d. h. beispielsweise Polyethylen-, Polypropylen-, Polyvinylchlorid oder Polyesterfolien. Als besonders geeignet haben sich Folien auf PolyethylenBasis erwiesen, wie beispielsweise eine MehrlagenVerbundfolie aus den Schichten Polyethylen/Polyvinylacetat/Polyvinylidenchlorid/Polyvinylacetat/Polyethylen.

Die Verbindung zwischen dem Trägermaterial und diesen Folien kann auf beliebige Weise hergestellt werden, d. h. in erster Linie durch Verklebung oder Verschweißung mittels Wärmekontakt, Hoch- oder Radiofrequenz, wobei letztere Verfahren bevorzugt sind. Wesentlich ist dabei, daß die Verbindung zwischen den beiden Flächen nur stellenweise vorgenommen wird, so daß zwischen den einzelnen Verbindungsstellen durchgehende Kanäle von den inneren Bereichen der Fläche zu ihrem Umfang offen bleiben und dadurch ein Luft- und Wasserdampfaustausch stattfinden kann.

Die durch die Verschweißungsstellen verschlossene Fläche des Trägermaterials soll etwa 5-60 % der gesamten Fläche betragen, um einerseits eine genügende Stabilität des Verbundproduktes und andererseits eine ausreichende Luft- und Wasserdampfdurchlässigkeit zu gewährleisten. Besonders günstig ist es, wenn die Verbindungsstellen nur etwa 10-20 % der Fläche ausmachen.

Die Verbindungsstellen, die mehr oder weniger gleichmäßig über die Fläche der Vorrichtung verteilt sein können, vorzugsweise jedoch in deren Randbereich, sind vorteilhafterweise punkt- oder stegförmig ausgeführt. Um die mittige Öffnung für das Stoma herum müssen die Folie und das Trägermaterial jedoch durch eine geschlossene Schweißzone (schweißring) miteinander verbunden sein, damit keine Ausscheidungen zwischen die beiden Schichten und dann nach außerhalb dringen können. Um dem Zug auch eines gefüllten Beutels sicheren Halt bieten zu können, ist es außerdem vorteilhaft, einen durchgehenden Verbindungsstreifen am oberen Rand der Vorrichtung anzubringen.

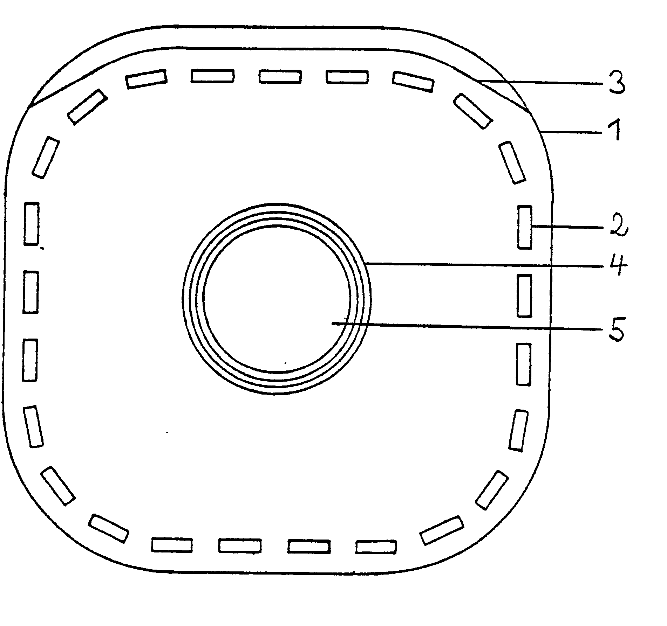

Die Erfindung wird nachstehend anhand des in der Zeichnung dargestellten Ausführungsbeispiels näher erläutert.

Die Abbildung zeigt etwa maßstabsgetreu die erfindungsgemäße Vorrichtung 1 von der Oberseite, d. h. von der Folienseite, her. Dabei bedeuten 2 stegförmige Verschweißungsstellen, 3 eine streifenförmige Verschweißungsstelle, 4 die ringförmige geschlossene Schweißzone und 5 die Öffnung für das Stoma. Paßgenau unter der Folie befindet sich das (nichtabgebildete) poröse Trägermaterial, das auf seiner Außenseite mit einer Selbstklebebeschicht versehen ist.

Die Vorteile der neuen Vorrichtung bestehen in der Kombination der von modernen, porösen Pflastern her bekannten guten Hautverträglichkeit mit der Möglichkeit eines beliebig häufigen Beutelwechsels während der mehrtägigen Verklebungsdauer. Ein weiterer Vorzug liegt in ihrem Tragekomfort. Während nämlich die bekannten Hautschutzplatten mit einem Materialgewicht von 800-1 500 g/m² relativ dick und steif sind, werden gemäß der Erfindung nur zwei dünne Materialien benötigt, die zuzüglich Klebemassenschicht etwa 200 g/m² wiegen. Die Hautschutzvorrichtung ist dadurch besonders flexibel und kann sich jeder Körperunebenheit und den Bewegungen der Haut voll anpassen, wohingegen die gebräuchlichen Platten aufgrund ihrer dicken Beschaffenheit sperrig und vom Benutzer stets zu spüren sind.

Aufgrund ihrer Hautfreundlichkeit und Anpassungsfähigkeit kann die erfindungsgemäße Vorrichtung außer zur Befestigung von Auffangbeuteln für die Versorgung von Stomata auch als Hautschutz unter jeglicher anderer Art von Drainageauffangmitteln, z. B. an stark sezernierenden Wunden, eingesetzt werden. Größe und Ausgestaltung der Vorrichtung müssen dann nur dem jeweiligen speziellen Verwendungszweck angepaßt werden.

Die Herstellung der erfindungsgemäßen Vorrichtungen wird im Folgenden beispielsweise beschrieben.

Beispiel 1

Auf ein 100 g/m² schweres, silikonisiertes Kraftpapier wird in einer Stärke von ca. 50 g/m² Trockengewicht aus einem organischen Lösungsmittel eine 30 %ige Polyacrylat-Selbstklebemasse, die durch Copolymerisation von 49 Gew.-Tln. 2-Äthylhexylacrylat, 49 Gew.-Tln.n-Butylacrylat und 2 Gew.-Tln. Glycidylmethacrylat hergestellt wurde, ausgestrichen und getrocknet. Die Trocknung wird so durchgeführt, daß in der Klebemasse eine Vielzahl von Bläschen entsteht. Auf die getrocknete Schicht wird ein Vlies aus Polyesterfasern (ein nach dem « spun-laced »-Verfahren hergestelltes Polyestervlies, welches unter der Handelsbezeichnung « Sontara » als Type 8000 (41 g/m², vollflachig) bzw. als Type 8010 (44 g/m², mit Lochstruktur) erhältlich ist) aufkaschiert und anschließend werden durch Anwendung von Druck in einem Kalanderspalt die Bläschen zerstört, wobei sich poröse bis mikroporöse Poren in der Selbstklebeschicht bilden. Danach wird die Klebemasse thermisch etwa 1 Minute lang bei 125 °C vernetzt. Das Material wird dann auf der klebschichtfreien Seite mit einer MehrlagenVerbundfolie aus den Schichten PE/PVAc/PVDC/PVAc/PE (Handelsname : Saranex® 14,Dca. 65 µm dick) eingedeckt und mit den entsprechend geformten Werkzeugen, wie in der Abbildung mustermäßig gezeigt, nach dem HF-Verfahren miteinander verschweißt, gleichzeitig oder anschließend in der gewünschten Form ausgestanzt und für den Verkauf konfektioniert.

Beispiel 2

Das selbstklebende, poröse Trägermaterial wird, wie im Beispiel 1 beschrieben, hergestellt, jedoch anstatt des Polyestervlieses wird ein Vlies aus Polyethylen (Handelsname : Tyvek®, Type 1622 E, porös) verwendet. Als abdeckende Folie wird eine ca. 70 µm dicke Polyethylenfolie aus LDPE eingesetzt und diese über Wärmekontakt bei ca. 130 °C mit dem Vlies verschweißt. Die Verschweißungsstege werden dabei, abgesehen von dem Ring um das Stomaloch, strahlenförmig angeordnet.

Beispiel 3

Auf ein 100 g/m² schweres, silikonisiertes Kraftpapier wird in einer Stärke von ca. 50 g/m² Trockengewicht aus einem organischen Lösungsmittel eine 30 %ige Polyacrylat-Selbstklebemasse, die durch Copolymerisation von 49 Gew.-Tln. 2-Äthylhexylacrylat, 49 Gew.-Tln. n-Butylacrylat und 2 Gew.-Tln. Glycidylmethacrylat hergestellt wurde, ausgestrichen, getrocknet und thermisch vernetzt.

Auf die getrocknete Klebemasse wird die im Beispiel 1 beschriebene PE-Verbundfolie — nach vorheriger Corona-Druckvorbehandlung — mit der vorbehandelten Seite aufkalandert und dadurch im Übertragverfahren beschichtet. sodann wird nach bekannten Verfahren an einer Perforierwalze von der Folienseite her das Material porös gemacht, so daß es eine Luftdurchlässigkeit von $15 \pm 5$ cm³ . cm⁻² . sec⁻¹ aufweist.

Auf die nichtklebende Rückseite der perforierten Folie wird anschließend eine Folie der gleichen Art geschweißt, wobei die Verschweißung in diesem Fall sowohl nach dem HFals auch nach dem Wärmekontakt-Verfahren erfolgen kann.

### Beispiel 4

Es wird wiederum eine Vorrichtung aus 2 gleichen Folien hergestellt, von denen die auf die Haut zu klebende Folie durch Perforieren porös gemacht wird. Als Folienmaterial werden LDPE-Folien gemäß Beispiel 2 verwendet. In diesem Fall kann die Verschweißung nur nach dem Wärmekontakt-Verfahren ausgeführt werden.

### Patentansprüche

1. Selbstklebend ausgerüstete Vorrichtung (1) in Blattform mit einer vorzugsweise mittigen Öffnung (5) für medizinische Zwecke, welche aus einem porösen, luftund wasserdampfdurchlässigen Trägermaterial besteht, das auf der einen Seite mit einer Selbstklebemasse porös beschichtet ist, dadurch gekennzeichnet, daß sie auf der anderen Seite mit einer etwa gleich großen Folie abgedeckt und mit dieser stellenweise in unterbrochener Anordnung verbunden ist.

2. Vorrichtung gemäß Anspruch 1, dadurch gekennzeichnet, daß die Verbindungsstellen insgesamt etwa 5-60 %, vorzugsweise 10-20 %, der Fläche der Vorrichtung (1) ausmachen.

3. Vorrichtung gemäß Anspruch 1 oder 2, dadurch gekennzeichnet, daß das poröse Trägermaterial aus einem Vlies aus Natur- oder Kunststofffasern, vorzugsweise Polyesterfasern, besteht.

4. Vorrichtung gemäß Anspruch 1 oder 2, dadurch gekennzeichnet, daß das poröse Trägermaterial aus einer perforierten Folie besteht.

5. Vorrichtung gemäß Anspruch 1, 2, 3 oder 4, dadurch gekennzeichnet, daß die Abdeckfolie ganz oder teilweise aus Polyethylen besteht.

6. Vorrichtung gemäß einem oder mehreren der Ansprüche 1-5, dadurch gekennzeichnet, daß das poröse Trägermaterial und die Abdeckfolie durch punkt- und/oder stegförmige Verschweißungsstellen (2) miteinander verbunden sind.

7. Vorrichtung gemäß einem oder mehreren der Ansprüche 1-6, dadurch gekennzeichnet, daß das poröse Trägermaterial und die Folie um die mittige Öffnung (5) herum durch eine geschlossene Schweißzone (4) miteinander verbunden sind.

8. Vorrichtung gemäß einem oder mehreren der Ansprüche 1-7, dadurch gekennzeichnet, daß das poröse Trägermaterial und die Folie am oberen Rand der Vorrichtung durch einen durchgehenden Streifen (3) miteinander verbunden sind.

9. Vorrichtung gemäß einem oder mehreren der Ansprüche 1-8, dadurch gekennzeichnet, daß die Selbstklebeschicht mit einem klebstoffabweisenden Trägermaterial abgedeckt ist.

### Claims

1. Self-adhesively finished device (1) in sheet form with a preferably central opening (5) for medical purposes, which consists of a porous carrier material which is permeable to air and water-vapour and which is coated porously on one side with a self-adhesive composition, characterized in that it is covered on the other side by a film of approximately the same size and is joined to the latter at spots in an interrupted arrangement.

2. Device according to claim 1, characterized in that the joining spots in total take up about 5-60 %, preferably 10-20 %, of the surface area of the device (1).

3. Device according to claim 1 or 2, characterized in that the porous carrier material consists of a nonwoven of natural or synthetic fibres, preferably polyester fibres.

4. Device according to claim 1 or 2, characterized in that the porous carrier material consists of a perforated film.

5. Device according to claim 1, 2, 3 or 4, characterized in that the cover film consists wholly or partially of polyethylene.

6. Device according to one or more of claims 1-5, characterized in that the porous carrier material and the cover film are mutually joined by welds (2) in the form of spots or webs.

7. Device according to one or more of claims 1-6, characterized in that the porous carrier material and the film are mutually joined around the central opening (5) by a sealed welding zone (4).

8. Device according to one or more of claims 1-7, characterized in that the porous carrier material and the film are mutually joined at the upper edge of the device by a continuous strip (3).

9. Device according to one or more of claims 1-8, characterized in that the self-adhesive layer is covered by a non-stick carrier material.

### Revendications

1. Dispositif équipé auto-adhésif (1) sous forme de feuille avec un orifice de préférence central (5) pour usages médicaux, qui consiste en un matériau support poreux qui est perméable à l'air et à la vapeur d'eau et qui est revêtu de manière poreuse sur un côté d'une masse autoadhésive, caractérisé en ce qu'il est couvert de l'autre côté d'un film ayant approximativement les mêmes dimensions et qu'il est relié à celui-ci de place en place en arrangement interrompu.

2. Dispositif selon la revendication 1, caractérisé en ce que les points de liaison occupent au total environ 5-60 %, de préférence de 10-20 %, de la surface du dispositif (1).

3. Dispositif selon la revendication 1 ou 2, caractérisé en ce que le matériau support poreux est constitué d'un non-tissé de fibres naturelles ou synthétiques, de préférence de fibres de polyester.

4. Dispositif selon la revendication 1 ou 2, caractérisé en ce que le matériau support poreux est constitué d'un film perforé.

5. Dispositif selon la revendication 1, 2, 3 ou 4, caractérisé en ce que le film de couverture est constitué entièrement ou partiellement de polyéthylène.

6. Dispositif selon une ou plusieurs des revendications 1-5, caractérisé en ce que le matériau support poreux et le film de couverture sont joints l'un à l'autre par des soudures (2) en forme de points ou de fins réseaux.

7. Dispositif selon une ou plusieurs des revendications 1-6, caractérisé en ce que le matériau support poreux et le film sont joints l'un à l'autre autour de l'orifice central (5), par une zone de soudure scellée (4).

8. Dispositif selon une ou plusieurs des revendications 1-7, caractérisé en ce que le matériau support poreux et le film sont joints l'un à l'autre au bord supérieur du dispositif par une bande continue.

9. Dispositif selon une ou plusieurs des revendications 1-8, caractérisé en ce que la couche autoadhésive est recouverte d'un matériau support qui n'adhère pas.